# EUROPEAN PATENT SPECIFICATION

(11) **EP 1 311 241 B1**
(45) Date of publication and mention of the grant of the patent: **14.01.2009**
(21) Application number: 01958246.9
(22) Date of filing: 21.08.2001
(51) Int. Cl.: A61K 9/14, A61K 9/16, A61K 9/20, A61K 47/02, A61K 9/00

(54) **USE OF A POROUS CARRIER**
VERWENDUNG EINES PORÖSEN TRÄGERMATERIALS
UTILISATION D'UN SUPPORT POREUX

(30) Priority: 21.08.2000 GB 0020610
(43) Date of publication of application: 21.05.2003
(73) Proprietor: DYTECH CORPORATION LIMITED, Dronfield Sheffield S18 1LS (GB)
(72) Inventor: SAMBROOK, Rodney, Martin, Dytech Corporation Ltd., Sheffield, South Yorkshire S18 1LS (GB); AUSTIN, Wayne, Dytech Corporation Ltd., Sheffield, South Yorkshire S18 1LS (GB); SAMBROOK, Mark, Rodney, Dytech Corporation Ltd., Sheffield, South Yorkshire S18 1LS (GB); HANNON, Michael, Univ. of Warwick, Chemistry Dept., Gibbet Hill Road, Coventry CV4 7AL (GB)
(74) Representative: Moore, Christopher Mark
(86) International application number: PCT/GB2001/003739
(87) International publication number: WO 2002/015881

(56) References cited:
- EP-A- 0 758 633
- WO-A-99/16478
- US-A- 4 218 255
- US-A- 4 293 540
- DATABASE WPI Section Ch, Week 198436 Derwent Publications Ltd., London, GB; Class A96, AN 1984-222577 XP002195310 & JP 59 131346 A (NGK SPARK PLUG CO LTD), 28 July 1984 (1984-07-28)

## Description

The invention relates to the uses of a porous carrier.

In our patent EP0598783B (Agents ref: P00914PCT(EP)) we have described and claimed:
"a method of making a porous refractory article composed of refractory particles, the method comprising the steps of:
   a) forming a dispersion comprising particles in a liquid carrier;
   b) introducing gas into the dispersion;
   c) removing the liquid carrier to provide a solid article having pores derived from the bubbles;
   d) drying; and
   e) firing
characterised in that the dispersion contains a polymerisable monomeric material."

In our patent application WO98/15505 (Agents ref: P01885PCT) we have described and claimed:
"a method of making a porous article composed of bonded particles (such as hydroxyapatite or the like) the method comprising the steps of:
   a) forming a dispersion comprising a liquid carrier and the particles and a polymerisable monomeric material;
   b) forming a foam of the dispersion;
   c) polymerising the foamed structure;
   d) drying the structure to remove the liquid carrier and provide a solid article having pores derived from the bubbles, and
   e) firing the article to remove the organic binder and provide a ceramic bond
characterised in that small bubbles of gas are introduced in the dispersion with agitation to form the foam and are allowed to cause to coalesce before the polymerisation of the monomeric material.

More specifically we have described and claimed in WO98/15505:
"a method of making a porous article composed of bonded particles the method comprising the steps of:
   a) forming a dispersion comprising a liquid carrier and the particles and a polymerisable monomeric material;
   b) forming a foam of the dispersion;
   c) polymerising the foamed structure;
   d) drying the structure to remove the liquid carrier and provide a solid article having pores derived from the bubbles, and
   e) firing the article to remove the organic binder and provide a ceramic bond
      characterised in that small bubbles of gas are introduced in the dispersion with agitation to form the foam and are allowed to cause to coalesce before the polymerisation, and in that the firing is carried out at a temperature appropriate to the growth of bone cells."

In our patent application GB 0009731.1 (our ref: P02810GB) we have described and claimed a method of making a ceramic foam by extrusion under low pressure. The foam ceramics made by that method are useful in the present invention.

US 4,293,540 and JP59131346 disclose implantable porous ceramic materials used for in situ drug release. WO99/16478 discloses ceramic bone substitute materials which may contain a drug.

It has now surprisingly been discovered that the structure of a porous carrier made by methods according to the earlier applications makes the carrier particularly suitable for carrying a wide range of materials and provides control on how the carried materials may be released. The porous carrier according to the invention is as defined in the claims.

According to this invention in one aspect there is provided a preformed porous ceramic carrier comprising an interconnected skeleton having pores the majority of which are in the range of from about 20 to about 1000 micron, the carrier having a density of less than 40% theoretical, the pores containing a second material therein, the rate of release of the second material from the carrier being controlled.

In accordance with this invention, the carrier comprises a preformed porous ceramic network comprising an interconnected skeleton having pores the majority of which are in the range of about 20 micron to about 1000 micron and a density less than 40% theoretical. The skeleton is made up of scaffolding and struts. The pore size distribution is controllable with average pore sizes in the range of 50 to 800, preferably 60 to 650 micron. The pore size is optimised to meet specific applications. For example, for general cell infiltration and vascularisation a carrier having pores in the range of about 50 to about 1000 micron is suitable, whereas for bone cell ingrowth pores within the range of about 100 to about 500 microns are preferred. The pores size required for the deposition of degradable materials within the pores will require a larger pore size to accommodate the thickness or deposition of a layer. Such a carrier may be made by a method according to the above cited patents and patent applications.

The carrier has a substantially totally interconnected porosity at densities less than 30% of theoretical density. The density may range from about 10% to about 40%, preferably about 30%. If the theoretical density is less than 10% of theoretical then a lack of strength is observed and the carrier becomes friable. If the theoretical density exceeds 40% the interconnectivity may not be total and the pore sizes may not be achieved. The density is determined by physical measurement of mass and volume. The theoretical value is taken from literature.

The ceramic carrier may be made from particles such as oxides and non-oxides. These materials are inherently stable to water or have a surface coating which is stable to the process conditions. Materials which have been used include alumina, zircon, spinel, silicon carbide, tin oxide, NZP, hydroxyapatite or other derivations of calcium phosphate, zirconia, kyantie, cordierite and the like. The ceramic may be fully or partially resorbable, when the carrier is used for medical purposes. The resorption process involves elimination of the original implant materials through the action of body fluids, enzymes or cells. Resorbed calcium phosphate may, for example, be redeposited as bone mineral, or by being otherwise reutilised within the body, or excreted.

Preferably the preformed porous ceramic carrier has a controlled degree of retriculation. The retriculation should be high to reduce the pressure gradient generated in infiltration and to minimise the level of defects associated with differential thermal contraction on cooling.

The density of the foam ceramic is preferably below 30% to ensure substantially totally interconnected porosity. Higher densities may be useful in circumstances in which a denser material is required for reasons of strength or permeability.

These denser materials, i.e. density higher than 30% of theoretical density but in the case of the foaming technique based on agitation limited to a maximum of 60% may be applied to the less dense materials in the green or fired state to create porosity gradients across the porous article. Before use, the article will need to be fired. The thickness of these layers may be varied to suit the application.

Higher density layers up to fully dense may be applied to the foam ceramic in the green state by processing techniques such as gel casting, coagulation casting. The article formed will need to be fired before use.

The proportions of the two phases may readily be adjusted so that the foam ceramic makes up the major component by volume of the formed body or the second phase, e.g. a metal phase does so.

The totally interconnected pore structure allows deep penetration of the pores of the carrier. The penetrating material may form a separate continuous matrix or it may be simply deposited on the interior walls.

The material to be introduced into the pores may be selected from a wide variety of materials. These include growth factors, such as human growth hormone or morphogenetic proteins; macrophages; antibiotics such as penicillin, tetracycline, mystatin; vitamins such as vitamin D, proteins such as polypetides, proteins; hormones, and the like. Specific materials include:
- a bone growth material
- FGF (fibroplast growth factor)
- IGF-1
- IGF-II
- PDGF (platelet derived growth factor)
- TGF-B (transforming growth factor)
- BMP-Z (bone morphogenetic protein)
- HGH (human growth hormone)
- Concentrations of human derived growth factors

Other examples include at least one cell which is a bone-forming or bone-degrading cell. Particularly useful cell types include chondrocytes, osteocytes, osteoblasts, osteoclasts, mesenchymal stem cells, fibroblasts, muscle cells, hepatocytes, parenchymal cells, cells of intestinal origin, nerve cells, and skin cells, and may be provided as primary tissue explants, preparations of primary tissue explants, isolated cells, cell lines, transformed cell lines, and host cells. The material may also be a chemotherapy agent or a radio opacifying agent. A number of sustained release anti-cancer drug delivery systems employing biomaterial carriers such as carbon particle, ethyl ester of iodinated poppy seed oil fatty acids and fibrin clot have been developed to deliver chemotherapeutic agents at high concentrations for long periods of time and reduce the systemic side effects, and these may be incorporated too.

The invention can thus provide a means for the introduction of a bio-compatible ceramic into the human or animal body to provide a localised source of drugs at a controlled rate of release to provide more effective treatment of various diseases. In particular the invention allows more effective treatment of cancers and tumours and minimises side effects by localising the treatment to specific sites within the body for treatment by chemotherapy agents such as cisplatin or radiography treatment by radioactive agents such as strontium - 67 or samarium - 153. In some cases these agents are preferred fixed within the pores.

Osteosarcoma is a malignant bone tumour in which normal bone tissue is destroyed and neoplastic osteoid is produced by abnormally proliferating, spindle cell stroma. It is a common primary malignant tumour of the bone. Adjuvant chemotherapy, i.e. surgery followed by chemotherapy, such as with antimetabolites, helps to prevent micro metastatic tumours and local reoccurrence whilst allowing continued bone growth as a result of their selectivity towards neoplastic cells. The antimetabolites are agents that interfere with the normal metabolism due to their structural similarity with normal intermediates in the synthesis of RNA and DNA precursors. They either serve as substrates for enzymes, inhibit enzymes, or do both. Due to differences in metabolism between normal cells and cancer cells, several antimetabolites have the potency to act with a certain degree of specificity on cancer cells.

Methotrexate (MTX), the 4-amino, 10-methyl analogue of folic acid, remains the most widely used antifolate in cancer chemotherapy, with documented activity against leukaemia, breast cancer, head and neck cancer, lymphoma, urothelial cancer, choriocarcinoma and osteosarcoma. This class of agents represents the best characterised and most versatile of all chemotherapeutic drugs in clinical use. MTX is a tight binding inhibitor of dihydrofolate reductase((DHFR), a critical enzyme in maintaining the intracellular folate pool in its fully reduced form as tetrahydrofolates.

MTX is most active against rapidly proliferating cells, because the cytotoxic effects occur primarily during the S phase of the cell cycle. During longer drug exposures, more cells are allowed to enter the DNA synthetic phase of the cell cycle, resulting in greater cell kill. In addition, MTX polyglutamate formation is substantially enhanced with longer periods of drug exposure, thereby increasing cytotoxicity. The cytotoxic effects of MTX are also greater with increasing drug concentrations. Therefore, MTX cytotoxicity is highly dependent on the absolute drug concentration and the duration of exposure. Thus, administration for osteosarcoma has been used at high doses (1-33g/m2) since the early 70's.

The carrier is one which has biocompatibility, i.e.
"the ability of a material to perform with a specific biological activity or, elicit an appropriate response in order to achieve a fully sophisticated and efficient function for a specific application"

The biomaterial must achieve acceptance, biological recognition, and adequate incorporation and be bioactive rather than absolutely inert. This has lead to the development of the 'second generation' biomaterials that are being specifically designed with the target that the biomaterial should elicit a positive specific response from specific cells and tissues at the implant site within the body. Thus, they may enhance the stimulation of osteoblasts in order to rapidly deposit mineralised bone matrix upon the surface or in close apposition to newly implanted prostheses and achieve better osseointegration. One such biomaterial that possesses these properties is hydroxylated calcium phosphate ceramic or hydroxyapatite (HA).

HA is a biocompatible, bioactive material that elicits a low immunogenic response. HA also possesses osseoconductive properties i.e. the ability to encourage bone growth directly along or towards its surface when placed in the vicinity of viable bone or differentiated bone forming cells. Furthermore, it may allow growth of an advancing edge of healing callus by providing a mechanically supportive lattice onto which new bone may grow. It has long been established that the crystal structures of synthetic HA and the inorganic component of bone, bone mineral are amazingly similar and a nonmechanical bond of significant strength has been shown to be formed between HA and bone. This characteristic has led to great interest in its potential use as a biomaterial for the repair of osseous defects as it allows direct chemical bonding of bone i.e. promotes bone deposition onto the surface.

Despite these interesting and useful properties the biomechanical mismatch and poor mechanical performance of these materials remain, due to the poor load bearing capacity of HA. Thus, clinical utilisation has been limited to applications where calcium phosphate ceramics confer bioactivity onto near inert implants (e.g. coatings on metal prostheses) or in defects that occur in less weight bearing areas e.g maxillofacial application.

In addition to these bone-enhancing properties, HA is able to readily adsorb biological factors and possibly chemical compounds onto its surface. Depending on the structure of the molecule and the surface chemistry of the particular HA, this adsorption may occur by physical adsorption and chemical adsorption. The amount of the chemical adsorbed may correspond to several mono layers on the surface.

HA may attain superior bioactive properties with its presence alone and in combination with tissue influencing agents adsorbed onto its surface to be released in the body at therapeutic concentrations e.g. antibiotic and anticancer agents. Their target applications would be to encourage restoration and repair of tissue function (e.g. of bone tissue) and to develop a bioactive interaction in a stable equilibrium state (e.g. enhanced ossointegration) by maintaining the natural activity of surrounding cells and tissue in their environment (e.g. of bone cells), whilst at the same time destroying unwanted tissues such as neoplastic or, bacterial cells.

It is a feature of this invention that when HA is presented as a carrier defined above the required advantages are obtained.

The aim of a drug delivery system is to achieve an optimal local dose at a specific desired site. Conventionally, drug administration (either orally or, intravenously) are at zones remote from their target tissue and thus, drug level and duration of the bioavailability cannot be controlled independently and the drug is free to diffuse throughout the body which may give rise to systemic problems and complications. These systems are aimed at increasing the bioavailability of drugs and factors and hence allow a more efficient and controlled action over time at specific sites. Controlled release has often been equated to a biphasic release pattern where a rapid release is achieved during an early phase followed by a slower sustained release during the late phase.

Experiments conducted to date as part of this invention have show the effectiveness of HA for delivering antibiotics and treating bone after curettage of infected bone. Chemotherapeutic agent loaded HA blocks could be useful to fill grafts after the curettage of bone tumours as well. This system will achieve the optimum effects of chemotherapy by exposing the tumour to a high concentration of an anticancer agent for long periods of time to reduce the proliferation of and kill all local tumour cells. It is postulated, according to this invention, that localised and sustained release may be achieved more easily by the use of interlinked porous HA structure having the ability to adsorb substances to its surface. The porous structure allows a greater surface area for the drug to adsorb onto and thus released into the body by desorption. The drugs may be any of those listed above, and others, e.g. poly(lactic/glycolic acid) polymers, PEMfTHFMA, gelatin and fibrin glue.

Local administration via HA is feasible and effective for the local control of bone and soft tissue tumours, for reasons of safer administration of the drug, easier assessment of chemotherapeutic effect, prevention of local reoccurrence and low frequency of systemic side effects.

In addition, an HA carrier according to the invention exhibits excellent biocompatability and similar mechanical and chemical properties to bone allowing tissue incorporation and vascularisation. It confers osseoconductive and osseoinductive properties acting as a bone graft or, implant coating avoiding the need for donor sites, which frequently causes patient morbidity. These properties allow osteoblasts to migrate and grow into the porous coating of the implant, creating an interlocking bond which limits motion between the implant and bone and therefore enhancing prosthetic stability, whilst the released drug performs its chemotherapeutic action.

Hence, according to the invention, HA impregnated with chemotherapeutic agents may be used after tumour surgery to fill defects in non-weight bearing areas such as in the frontal, occipital lobes of the skull, the maxilla and mandible of the face or, in weight bearing areas when incorporated onto a prosthesis in limb salvage therapy.

MTX may be used as the anticancer agent in HA blocks due to its proven efficacy against osteosarcoma, ease of detection in comparison to other anticancer agents and its relative selectivity towards neoplastic cells. As well as examining the elution kinetics of a HA-MTX complex we have also assessed the cytotoxic effects of a raised concentration and exposure time (above the systemically administered duration of 24 to 42 hours) on osteoblast cells and two ostoesarcoma cells in order to assess if an increased exposure time and concentration of MTX would be more effective in treating ostosarcoma but not affect the proliferation of osteoblasts. This was studied because MTX is one of the few chemotherapeutic agents where studies have indicated selectivity to neoplastic cells, a concentration-effect and time-effect relationship. The idea of this MTX-HA system is to provide increased concentration and exposure time with a minimum effect on other tissue thus; these properties of MTX must be shown in order validate the HA- MTX system for potential use in clinical practice.

The carrier may contain in its pores materials other than the drugs listed above. Such other materials include Wemer-type coordination complexes (e.g. cobalt (III) hexammine salts, iron (II) tris(phenanthroline) salts, cis-platin, carboplatin and oxaliplaten, complexes of edta), macrocyclic complexes (e.g. metallo-porphyrins), metallocenes and sandwich complexes (e.g. ferrocene and titanocene) and organometallic complexes (e.g. methylcobalamin).

Preferably, the control is arranged to slow down the rate of release.

The control is achieved by placing the second material in a degradable support, e.g. a biodegradable support such as collagen or a polymer or the like. Specific biodegradable polymers in the context include PCPP.SA (poly(carboxy phenoxy) propane-sebacic acid, PCC, CPP.SA, FAD-SAPTMC, PAA, and the like. In general terms the polyanhydrides, polyorthoesters, polylactides and polyglycolides and copolymers thereof are suitable.

The use of a degradable intermediate carrier is attractive because it is so versatile thus the deposit may be layered in different ways, e.g:
- alternating layers of agent-free resin or polymer and of agent containing layers;
- varying the concentration of agent across different layers of resin or polymer.

A carrier may be filled in a variety of ways using vacuum or pressure or both. These layers may be built up by simple sequential impregnation, incipient wetness or other techniques known to those skilled in the art, if a homogeneous distribution is required throughout the body. Other techniques can be used if a heterogeneous distribution is required. For example, by using a centrifugal impregnation technique a specific layer can be concentrated at the outer sections of the foam ceramic. Layers may be built up in a mechanical fashion by inserting pieces of foam with a specific agent or drug within a foam containing another agent or drug. Freeze drying is also preferred.

Drugs may be released at precise doses into the body. Either a biodegradable monomer incorporating known concentrations of the drugs will be used to impregnate the porous article and then polymerised or a polymer incorporating the drugs will be used to impregnate the porous article.

The external geometric surface and/or the surface of the inner pores may be coated with bio-degradable polymers incorporating drugs or more specifically anti-cancer agents. These polymers may fill the voids within the porous article and act as a reservoir for the slow release agents. As indicated, layers may be built up such that individual layers have different functions. For example, the first layer may contain growth factors, the second layer is pure polymer, the third layer may incorporate antitumour factors such as cisplatin. Each layer may have different rates of biodegradability, by changing the nature of the polymer, such that the release rates of the various agents are controlled and predictable.

While the article of this invention may be shaped for any use it is preferred that it be shaped for replacement as e.g.
orthopaedic
maxillo-facial, or
cranio-facial
or the like.

Such grafts may be used for example for:
- substitution of bone segments following surgery
- filling of large loss of bone, following trauma or infection
- repair or reconstruction of damaged joints

In addition, an article of the invention may be located in the body at other locations, e.g. intramuscular sites, interperitoneal sites, subcutaneous sites, central nervous system sites and occular sites.

The porous ceramic can be shaped such that inserts of dense ceramic such as alumina or perhaps metals can be put into place where perhaps a higher mechanical strength is required from the implant in ultimately load bearing situations. The ceramic or metal insert may be exposed at one or more of the foam ceramic external surfaces or be enclosed within a shell of foam ceramic. The thickness of this shell may typically be from 1 mm to 10 mm but is not limited to this range. Alternatively, the foam ceramic may be the insert contained within a dense ceramic or metal.

In order that the invention may be well understood it will now be described by way of illustration with reference to the following examples.

### EXAMPLE I

Porous HA materials manufactured by a method according to the invention that produced an interconnected porous ceramic with a maximisation of mechanical strength by introducing macropores into a densified matrix in accordance with the Dytech patents above. These are available under the trade mark **HI-POR**^{®}**.** No impurities are introduced during the forming process. Although the chemical composition of the finished product is the same as those HA products already on the market, it is produced by a technique which allows a unique physical structure. The porous HA specimens were manufactured as cylinders (approximately 13mm diameter by 6mm). One type of HA was used in the study consisting of a pore density of 18% and a mean pore size of 300µm.

The porous HA specimens alone are referred to as discs and once they are loaded with drugs such as MTX for delivery, they are referred to as 'systems'.

The MTX (David Bull Laboratories, Onco-Tain at 25mg/ml) was of the clinical preparation containing methotrexate B.P .25mg, sodium chloride B.P. 0.49% w/v sodium hydroxide. The MTX was stored at 4°C.

4ml of MTX at 25mg/ml was then mixed with 46m1 of Dulbecco's phosphate buffered saline (Sigma -D8537) and thoroughly mixed to obtain a homogenous mixture. Care was taken not to expose the MTX to direct light as this may alter its chemical composition.

48 HA units at 300J.Im pore size and 18% density were each placed into 7ml Bijou containers (Sterilin cat. No 1298) and 1ml of the MTX/PBS mixture was added to each UJlit aJld the Bijou capped. The fully submerged samples were then loaded in four different ways. The fully submerged samples were then loaded in four different ways.

### Simple absorption

The first method involved loading by simple absorption of MTX solution allowing passive interspersion through interconnecting pores. This was done by placing 18 discs into MTX solution in an incubator set at 37°C. 6 units were then removed after 1 hour in the MTX, the next 6 removed after 3 hours and the final 6 removed after 6 hours.

### Vacuum impregnation

Loading by vacuum was done by placing 18 units into a vacuum chamber (Angil Scientific, Cambridge England, no -1506) with the lids of the bijou containers loose. The vacuum was then switched on at 150mbars pressure, thus creating a negative pressure within the container and allowing the extrusion of the MTX through the macro and micro pores of the units. 6 samples were removed from the MTX after 1 hour in the vacuum, 6 units removed after 2 hours and 6 units after 4 hours.

### Centrifugation

This involved placing 6 units in their bijou containers with the lids tightly closed. Each bijou container was the placed into a Blue Max 50rn1 polypropylene conical tube (Becton Dickinson 30 x 115mm,). Each conical tube was tightly capped and placed into a centrifuge (Centra-3, Damon/IEC, Bedfordshire, England, no-23670102) at 1000rpms for 1 hour. Each conical tube was counterweighted by another sample contained in a conical tube.

### Freeze drying

This involved removal of the caps from the bijou containers of 6 units and placement into a freeze dryer (Modulyo, Edwards, model no -165005) at -60°C and 8mbar pressure. All the samples were placed for approximately 24 hours and removed. All systems were removed from bijou containers and allowed to air dry overnight under aseptic conditions.

Once all samples were dry the elution study could be conducted. This process involved the addition of the eluate, 5ml of Dulbecco's Modified Eagle's Medium (DMEM) and 5ml Dulbecco's Phosphate Buffered Saline (PBS). Each system was then gently placed into a Imiversal tube using tweezers to keep the samples sterile and prevent any sudden movement that may have shaken off any loaded MTX .Half the systems of each loading technique were each added to 5ml of medium and the other 14 and the other 24 to PBS.

The 48 systems were all then placed onto rollers, which were inclined in order to entirely submerge all the systems with the eluant. Systems were then removed under a sterile hood and by the use of sterile tweezers and placed into a new universal tube containing 5 ml of fresh eluant at 5 different time points -2hrs, 4hrs, 24hrs, 48hrs and 168hrs The 240 samples containing the released MTX were then analysed using UV spectrometry techniques.

### Quantification of MTX in eluation samples by UV Spectrometry

UV analysis of MTX in sample elutions were performed at 303nm using a Unicam UV4 spectrometer (Cambridge, UK). Firstly, a serial dilution of MTX from 1000µm/ml in PBS and DMEM was done in order to obtain a set of known concentrations of MTX in the drug release samples (Appendix III). Values taken were done down to the lowest concentrations that seem detectable by the UV spectrometer and values plotted were within the linear range of the calibration data.

The equations of the calibrations obtained were:
1) PBS_{1y} = 0.0664ₓ + 0.0044
2) Medium_{y} = 0.0663ₓ + 0.0074
where _{y} = absorbance reading (nm) and
ₓ = MTX concentration (µg/ml)

1ml solution containing Medium or PBS with no MTX was placed in a quartz curette and into the UV Spectrometer as a baseline value. 1ml of the eluants were then placed into another quartz cuvette and the absorbance reading measured against this baseline value. The absorbance readings were then placed into equations 1 or 2 to obtain the concentrations of MTX.

Some absorbance readings were higher than the linear portions of the calibration curves i.e. greater than 2.079nrn for PBS and greater than 1.040nrn for medium. Thus a 1 in 5 dilution was done on these samples to obtain readings on the calibration curve. These samples were mainly those which were taken after 2 hours. The results of the release concentrations of the systems using the 4 different techniques were then tabulated and the Tukey Kramer Honestly Significant Difference Test (TKHSDT) to determine any significant differences between the amounts existed.

### In Vitro Cell Culture

Two osteosarcoma cell lines and primary human osteoblasts were used in the cell culture studies of the effect of increased MTX concentration and duration time. The MG63 cells were obtained from a 14 year old caucasian male (ATCC- CRL-1427). The human osteosarcoma like cells(HOS) cells were obtained from a 13 year old caucasian female (ATCC- CRL-1543). The human osteoblast like cells(HOB) were isolated from patients undergoing total knee replacement. The cell lines were each cultured in complete Dulbecco's Modified Eagle's Medium (DMEM) (Gibco, UK) supplemented with 10% foetal calf serum (FCS) (Gibco, UK), non essential amino acids (1%), ascorbic acid (150~g/ml), L glutamine (0.02M), HEPES (0.01 M), penicillin (100units/ml) and streptomycin (100~g/ml), at 37°C and 5% CO2 in a 99% humidified sterile atmosphere. The medium was changed at least twice a week to prevent nutrient depletion-and waste product accumulation.

Once the cells had become confluent, they were removed by emptying the medium, washing with 10m1 PBS and adding 5m1 of 0.25% Trypsin and incubating at 37°C. After 5 minutes, the cell layer was removed from the tissue culture flash by tapping the flask onto a solid surface. The contents are then collected into a universal tube and the flask washed with 10ml medium, which is also placed in to the universal The container was then centrifuged at 2000rpm for 5 minutes at 18°C after which the trypsin-containing supernatant was removed, taking care not to dislodge the pellet of cells at the bottom of the container. Next the cells were counted under a light microscope and concentrations of 13.2 x 104 cells/ml made up. These were then seeded into 96 well plates. In total 6 plates, were seeded with 2 plates containing one of the three cell lines, for the purposes of measuring cell proliferation.

Each plate was allowed to incubate for 2 days and then 12 varying concentrations of MTX were added to each plate. Although dosages of MTX are given in mg/m2 BSA it was not possible to use such an index to calculate the appropriate dosage in this study. Therefore, it was necessary to ascertain the concentration that the doses would be in the plasma of an average man. This involved finding the proportion of the body weight that is fluid. The intravascular volume represented approximately 60% of the body weight. Therefore, as 1 kg is approximately equal to 1 litre, a 70kg patient has an intravascular volume of 42 litres. Therefore, the volume of fluid 1m² of BSA is:
42 litresll.73m2 = **24.3 litres**
   The BNF recommended dosage for single agent therapy of MTX (mg/m1 is 600.
   Thus the following plasma concentration is obtained in this therapy:
600mg/24300ml
   = 0.0247mg/ml
   **= 24.7µg/ml**

This initial concentration does fall as it is eliminated in the body, however, some studies conducted have used doses up to 2, 3 times this value. Thus, the concentrations used, ranged above and below 24.7g/ml ranging from 0.1µg/ml to 1000µg/ml. Three plates, one plate with each cell line, was given MTX for 24( one day) hours exposure and the other 2 plates given for 72(3 days) hours exposure to see the differences in cell proliferation with varying concentrations and increased exposure times.

### MTT Assay

The effects of MTX on the proliferation of ostersarcoma cells was measured by MTT assay. The reagents required for this assay were MTT powder (sigma M2128 or M5655), dimethyl sulphoxide DMSO (sigma D2650), and phosphate buffered saline (PBS). The medium was removed from the wells. The MTT solution was made by adding 50mg MTT to 10ml of PBS in a universal tube, which was warmed at 37°C for 15 minutes. The solution was then filtered and 10µ of MTT added to each well and returned to the incubator for approximately 3 hours at 37°C and 5% CO₂ and 99% humidity ( details on the function and action of MTT can be found in Appendix II). The MTT was removed from by inversion and blotting onto tissue paper. Next, 100µl of DMSO was added to each well and shaken for 1 minute. The absorbance of each well was then read by a Dynatech microplate reader (BioRad Model 3550) at a wavelength of 595nm and 5sec mixing time. These samples could not be re-used and were therefore discarded upon completion of the assay. Once readings were obtained they were tabulated and normalised as a percentage of the control value. Absorbance readings were analysed against the control values by a Dunnetts' test of significance at the 5% level in order to determine if there was a statistically significant difference in cell proliferation as concentration of MTX rose.

### RESULTS

### Evaluation of HA as a Drug Delivery System for MTX

The results showed that the different systems of porous HA loaded MTX successfully and released the anticancer drug. The differences in methods of loading had significant effects on the release kinetics for MTX. However, the results of the samples placed into medium provided erratic and unreliable absorbance readings by the UV Spectrometer and thus were not displayed graphically.

Overall a biphasic release pattern for controlled drug delivery was observed in systems 7 and 8, see Table 1 below. This involved a rapid early phase release with a sustained and slow but gradual late phase drug release. Systems 1 to 6 of Table 1 did not display this pattern and demonstrated a rapid initial deployment of MTX within the first 24 hours with an undetectable release in the late phase. The results for each system represent an average value of three replicates and the loading methods for each of the systems can be seen below on table 1.

**Table 1. Different techniques of loading MTX onto HA discs.**

| | |
|---|---|
| System 1 | Absorption for 1 hour |
| System 2 | Absorption for 3 hours |
| System 3 | Absorption for 6 hours |
| System 4 | Vacuum for 1 hour |
| System 5 | Vacuum for 3 hours |
| System 6 | Vacuum for 6 hours |
| System 7 | Centrifugation for 1 hour at 1000rpm |
| System 8 | Freeze drying for 24hours |

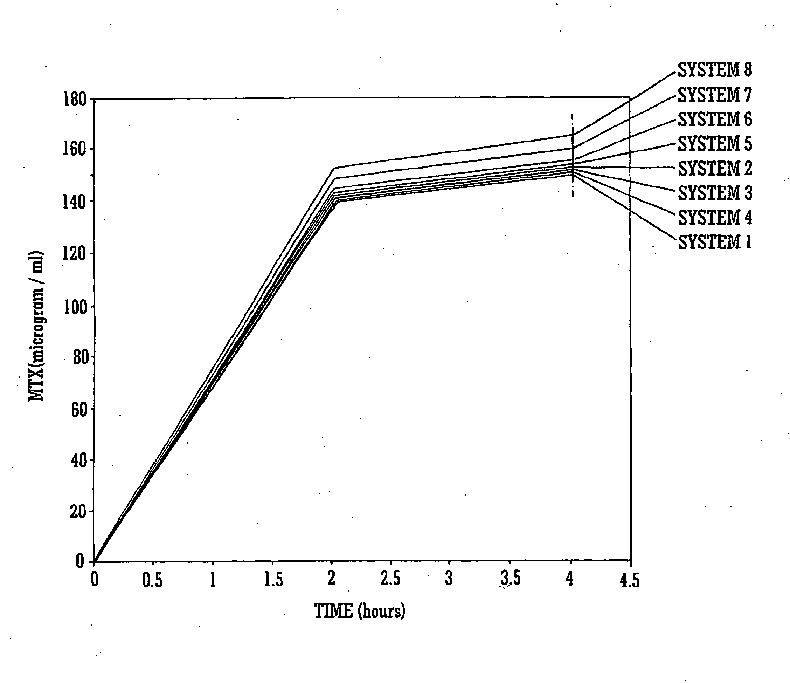

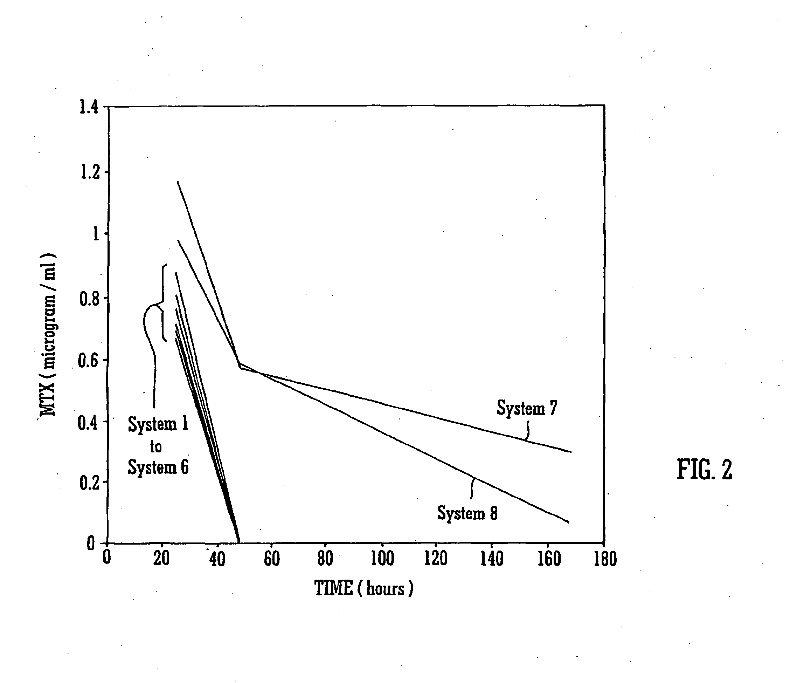

The results are shown in the graph of accompanying Figure 1 showing the initial release and in Figure 2 showing the release after 24 hours.

This study is significant in relation to a drug release system as MTX treatment systemically is normally only for 24 to 42 hours and close intensity studies have shown no difference in effectiveness as concentration is increased. This study aimed to see if the length of time that MTX is released from the drug delivery study is of sufficient duration and concentration to provide a greater therapeutic benefit by increasing osteosarcoma cell death. In addition the HOB cell line was tested to see if the choice of drug (MTX) could be selective enough for neoplastic cells and not inhibit bone growth by ostoblast cells.

Overall, the results allowed definition of optimally porous material systems based on positive release kinetic profiles. Elucidation of the optimal methods of loading were by centrifugation and freeze drying, which displayed the largest sustained release of all the systems. Furthermore, exposure time appeared to provide a significant decrease in cell proliferation of at least one of the osteosarcoma cell lines.

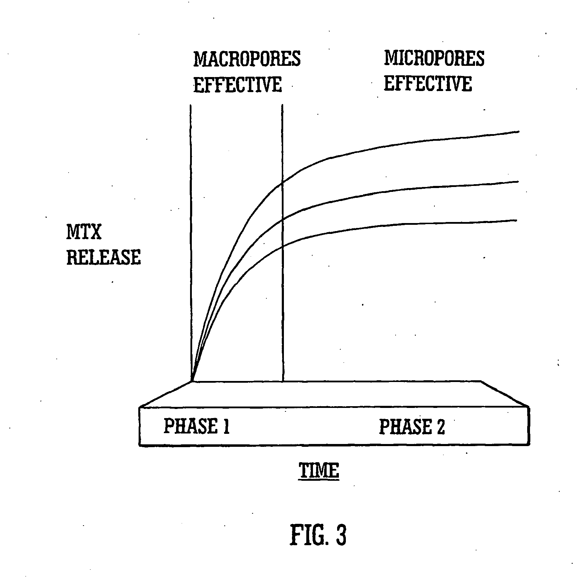

In general, the optimal profiles approximate to a 'biphasic model' of controlled release (Figure 3). This involves a rapid release during the early phase and gradual, sustained release during the late phase. These optimal profiles were the HA discs loaded with MTX by centrifugation (system 7) and freeze drying (system 8). These were seen as the optimal systems as they were the only ones to show a detectable release of MTX within the therapeutic range for up to 168 hours. Systems 1 to 6 (loaded by absorption and vacuum) showed a high initial release but no detectable release after 48 hours.

The release of a drug incorporated into the pores of porous hydroxyapatite has been attributed to the degree of macroporosity and microporosity with apparent Density (AD) reflecting the release profiles at the early stages as a function of macroporosity and Real Density reflecting the controlled release at the later stages as a function of the level of open microporisity. In other words, the percentage and size of macroporosity and the level of loaded drug carried (MTX) are dominant factors in the early stages and drug contained within micropores is the dominant factor in phase II late drug release.

The objective of this study was to determine the extent to which the different loading techniques could load the drug into the micro and macropores and hence obtain a sustained release. Since centrifugation and freeze drying provided a sustained late release as well as an early release, micro- and macropores must be loaded. A high early release is important in anti-cancer therapy as this can catch and kill early locally reoccurring cancer cells and a sustained late phase release is important to maintain the therapeutic effect. Thus, bioavailability of the MTX particularly with rapid early release and sustained late release may be essential in anti-cancer therapy.

All 8 systems tested showed a high initial release. This high initial release can be attributed to 'wash off' effect where solidified drug encrusted on the surface is dissolved when the system is initially placed into the first eluant. System 8 (Freeze Dry) showed the highest initial release after 2 hours and most likely attributed to the nature of the freeze drying process. At the end of the process samples have been concentrated, with solidified MTX encrusted onto the surface which may be partially tapped off but much excess MTX still remains and thus, this would be dissolved in the first eluant.

The highest rate of release after the first 4 hours was also exhibited by the freeze drying process (system 8). This was closely followed by the centrifugation process (system 7), then vacuum and the absorption(systems 1 to 6). This may have been attributed to the following reasons: The freeze drying process may provide immediate extrusion of the MTX solution into the pores of the HA unit allowing the MTX solution to enter the macro pores but the MTX is not in solution when the HA unit is removed. Thus, on removal of the HA disc some of the liquid MTX may leak out of the macropores in the other systems thus reducing the load of drug available for initial release. However, system 8 will not encounter leakage of the MTX from macropores on removal as it is already solidified within the pores.

The differences between initial release rates of systems 1 to 8 may be attributable to the ability of each method to allow efficient forceful extrusion of MTX through the macropores. Centrifugation was possibly the most forceful followed by freeze drying and vacuuming with the least forceful obviously being the simple absorption methods. Differences within increased vacuum times and absorption times are small with not much statistical differences shown by the TKHSDT, indicating that the maximisation of drug loading is obtained after the first time points i.e. after1 hour of absorption and 1 hour of vacuuming. The subtle differences may occur as a result of variation in pore size, density and pore interconnectivity within HA discs as a result of slight inconsistencies in manufacture.

The sustained release systems were systems 7 and 8 with system 7 providing a higher rate of late phase release. As mentioned late phase release is a function of microporosity .The fact that there was no release of MTX detected for systems 1 to 6 after 24 hours indicates either that the UV Spectrometer was insensitive to the small amounts of MTX or, that no MTX solution entered the micropores of the HA discs. It is however, likely that some MTX solution did enter the micropores and a small amount may have been released but was lower than the threshold for detection and possibly lower than the therapeutic threshold of 0.01 µm.

The rate of sustained late phase release is higher for centrifugation probably because the MTX solution was spun at 1000rpm for 1 hour allowing the micro pores to be filled with the drug more efficiently than the freeze drying technique. In addition, the ability to extrude a liquid into the micropores of HA may have been adequate under the negative pressure of the freeze dryer but the solution may have solidified before or, in mid process of entering the micropores.

Chemical forces of direct adsorption of the drug onto the surface may also play a role in early and late phase release. All these methods were without the use of a drug delivery carrier, such as gelatine or alginate, and the only ways in which the drug can stay bound on the HA surface is by physical and chemical bonds. Physically adsorbed MTX is accomplished by Van Der Waals forces. This allows several layers of the MTX molecule to bond to the surface of the HA. Chemical bonds are shorter in length and far stronger and consist of electrostatic (ionic) and/or, covalent bond types. This type of bonding only allows the formation of a monolayer of the molecule on the surface.

It is likely that the physically adsorbed drug on the HA surface contributes to early release as the temperature rises in the warm room (37°C) in which the systems are placed in that the physical bonds break releasing any drug in the first eluants. However, since chemical bonds are much stronger it is likely that they contribute to the late phase release to a greater extent. Since centrifugation seems to have provided the greatest coverage of micropores and hence, surface area, then it seems reasonable to suggest this resulted in a greater amount of coverage of the HA by physical and chemical bonds. Hence, the breakage of these bonds resulted in the highest rate and amount of release in the late phase of the study than any other system. These chemical bonds consist of covalent and ionic linkages but covalent interactions are unlikely to play a role in drug release as the strength of such bonds may not allow the MTX to desorb.. lonic bonds may be favourable for drug release and may occur between calcium ions and carboxl groups or between phosphate groupings and the N-methyl group of MTX. Other chemical bonds such as between hydroxyl groups of HA and aromatic nitrogens, carboxyl groups, amide/peptide bonds may also occur. Also various chelation type interactions between the calcium ions and the MTX molecule mediated via combinations of several or more of the following groupings present in the drug; amide/peptide groups, hydroxyl groups, aromatic nitrogens, free amino groups.

The significance of MTX being directly adsorbed onto HA is that HA coatings on an endoprosthesis in limb salvage surgery may be loaded with MTX thus, reducing the likelihood of local reoccurrence of osteo~arcoma.

The highest total accumulative release was displayed by system 8 and seems to correlate with the highest initial release after 2 hours. The amount of MTX released after 24 hours and onwards in proportion to the initial dose released is very small. Thus, the greatest total accumulative release in the systems would be largely governed by the initial amount released due to the 'wash off effect'.

The optimal method of loading HA discs with MTX was found to be by centrifugation followed by freeze drying. Other physical properties of the HA may determine its ability to act as a drug delivery system such as altered sintering times(which alters micropore interconnectivity), pore size and pore density. In addition to these studies the therapeutic effect of MTX after adsorption to HA should also be investigated as desorption may result in an altered chemical structure.

The effect of drug concentration and exposure time on the three cell lines-HOS, MG63 and HOB showed the following results. All of the cell lines after 1 day exposure showed a decrease in proliferation as MTX concentration rose. The least sensitive cell lines being the HOS cells, which showed only a small decrease in proliferation after addition of MTX and only 4 statistically significant differences out of the twelve MTX concentrations. After 3 day exposure at the same concentrations the HOBs and HOS cells showed a reduced level of cell proliferation compared with the 1 day exposure samples. Instead an erratic behaviour was shown by MG63 cells as concentration of MTX rose. Thus, these results show that ROS and ROBs are sensitive to an increased cytotoxic effect of increased exposure time of MTX. This may be explained by the fact that polyglutamate formation is substantially enhanced with longer periods of drug exposure, thereby increasing cytotoxicity. In addition it seemed that as exposure time lengthened the cytotoxic effect was minimally greater as concentration rose, suggesting exposure time is an important factor and perhaps a concentration threshold for cytotoxic effectiveness may have been reached.

MG63 was affected only slightly as MTX concentration and exposure time increased, which may be due to resistance. Although high dose MTX is frequently used in the treatment of osteosarcoma, conventional dose therapy may sometimes be ineffective. Several retrospective studies have suggested that a threshold peak MTX level needs to be achieved to obtain a good response to chemotherap/O-53. This relationship suggests osteosarcoma is intrinsically resistant to conventional doses of MTX that may be overcome by the use of extremely high doses. A potential mechanism for intrinsic resistance is decreased transport into cells via the reduced folate carrier (RFC), which could explain why exposure time had no effect as perhaps greater time is required to allow transport by alternative means, such as passive diffusion. In addition doses may not have been high enough to allow transport into cells. A second potential for intrinsic resistance is a result of impaired polyglutamylation, which leads to a lack of drug retention within the cell, inhibiting its action.

MTX is supposedly highly selective for neoplastic cells yet the HOEs were affected to a similar degree to the HOS cells. The reason for this is that MTX is most active against rapidly proliferating cells, because its cytotoxic effect primarily occurs in the S phase of the cell cycle. During longer exposures, more cells are allowed to enter the DNA synthetic phase of the cell cycle, resulting in greater cell kill. Since HOE cells cultured in vitro are rapidly dividing like the osteosarcoma cells they may be affected in the same way. This would simulate the clinical scenario where some osteoblast cells are not rapidly dividing. On the other hand in vitro studies have shown only a 30% decrease in proliferation of osteoblast cells compared to a 90% decrease in proliferation of osteosarcoma cells at 5mM MTX.

This investigation demonstrated that the porous HA was able to successfully release MTX for a period of 7 days using two of the methods of drug loading. In addition the levels of drug released were well within the therapeutic range for the treatment of osteosarcoma. Cell proliferation studies supported the therapeutic efficacy of an increased drug exposure time on the prevention of cell proliferation of HOS cells. The exposure time tested was longer than times allowed to administer the drug systemically and within the time period that the drug is released from the studied HA systems.

Thus, the application of MTX loaded HA as a drug delivery system for the treatment of osteosarcoma, shows great potential and may be used in the clinical scenario in non-weight bearing areas to fill small post operative defects, e.g. maxillo-facial surgery and may be incorporated onto a prosthesis in limb salvage surgery to treat load bearing areas.

### EXAMPLE II

### Absorbance of iron(II) tris phenanthroline perchlorate [Fe(phen)₃][C1O₄]₂ onto HA.

Four methods for the absorbance of [Fe(phen)₃][C1O₄]₂ onto HA were explored, each using blocks of porosity 83.35 (0006) and approximate dimensions 20 x 15 x 20 mm, weight 303.5 g. Solutions were prepared by dissolving [Fe(phen)₃][C1O₄]₂ (0.031 mmol) in methanol (30 cm³). All blocks were weighed wet and dried in an oven at approximately 100°C overnight.
1. Absorbance by immersion of HA in a solution of [Fe(phen)₃][ClO₄]₂ for 35 minutes.
2. Absorbance by immersion of HA in a solution of [Fe(phen)₃][ClO₄]₂ for 24 hours.
3. Absorbance by placing HA under vacuum for 10 minutes prior to the direct injection of a solution of [Fe(phen)₃][ClO₄]₂ onto the block and vacuum maintained for a further 30 minutes.
4. Absorbance by immersion of HA in a solution of [Fe(phen)₃][ClO₄]₂ and the application of a vacuum for 35 minutes.

The quantity of solution absorbed by the blocks ranged between 2.7-3.5 g, the greatest quantities were observed for the methods using vacuum. (The accuracy of weighing is limited due to solvent evaporation).

The observed penetration into the blocks was small in all cases with a large degree of surface absorption. The best penetration was observed for the blocks loaded according to method 2, i.e. prolonged immersion.

### Leaching of [Fe(phen)₃][ClO₄]₂ from HA blocks prepared according to method 3

Leaching with stirring of solutions to avoid inaccuracies due to concentration gradients. (According to present arrangements however some anomalies may result from occasional jarring of the block by the magnetic follower).

Half of the block prepared in method 3 was immersed in distilled water (30 cm3) and the absorbance recorded by UV-Vis spectrometry at wavelength 512 nm. An extinction coefficient of 10,620 mol⁻¹ dm³ cm⁻¹ was used in all calculations.

The graph below shows the quantity of [Fe(phen)₃][ClO₄]₂ released from the block over time. 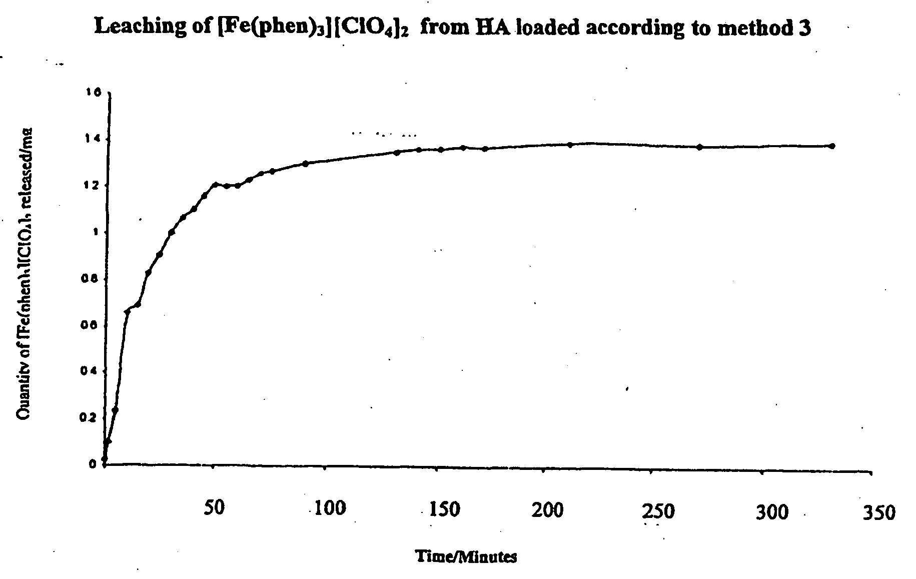 The graph shows two phases which are likely to correspond to the initial release of surface absorbed [Fe(phen)₃][ClO₄]₂ followed by release of [Fe(phen)₃][ClO₄]₂ from inside the block. Almost all [Fe(phen)₃][ClO₄]₂ is released from the block after 170 minutes. After 330 minutes the solution is replaced with distilled water and the absorbance measured after a further 20 hours. The reading revealed the release of a further 0.14 mg from the block, this may be due to the release of [Fe(phen)₃][ClO4]₂ which was inside the block when the system reached equilibrium during the first 330 minutes.

| | | | | | | |
|---|---|---|---|---|---|---|
| 50 | 100 | 150 | 200 | 250 | 300 | 350 |

### EXAMPLE III

### The use of Poly(DL-Lactide-co-Glycolide) to slow the release of [Fe(phen)₃][ClO]₂

Since the biodegradable polymer Poly(DL-Lactide-co-Glycolide) pLG) is not soluble in methanol acetonitrile was used for the following experiments.

### Loading

Vacuum method 3 was used for loading the compounds onto HA, again blocks of porosity 83.35% (0006) were used with approximate dimensions, 20 x 20 x 15mm, weight 3.6-3.8 g. For the control block with just [Fe(phen)₃][ClO₄]₂ (0.01 g, 0.013 mmol in 6 cm³ acetonitrile), good penetration into the block was observed. For the block loaded with [Fe(phen)₃][ClO₄]₂ (0.01 g, 0.013 mmol) and PLG (50:50) (200 mg in 6 cm³ acetonitrile) less penetration was observed but distribution was more uniform. Again the blocks were dried in the oven. (In order to fully dissolve the polymer in acetronitrile stirring for up to 1 hour is required before loading).

### Leaching

All leaching experiments were undertaken using the cut halves of the loaded blocks with distilled water (30 cm3) and constant stirring. Initial leaching experiments showed that the polymer impregnated blocks floated, necessitating weighting using glass rods. This buoyancy is likely to be due to the polymer blocking pores in the HA trapping air inside the block. In addition these experiments revealed that readings must be taken over a number of days hence a closed system is required to prevent evaporation of the water leading to inaccuracies. Again absorbance at λ 512 nm was recorded at appropriate intervals.

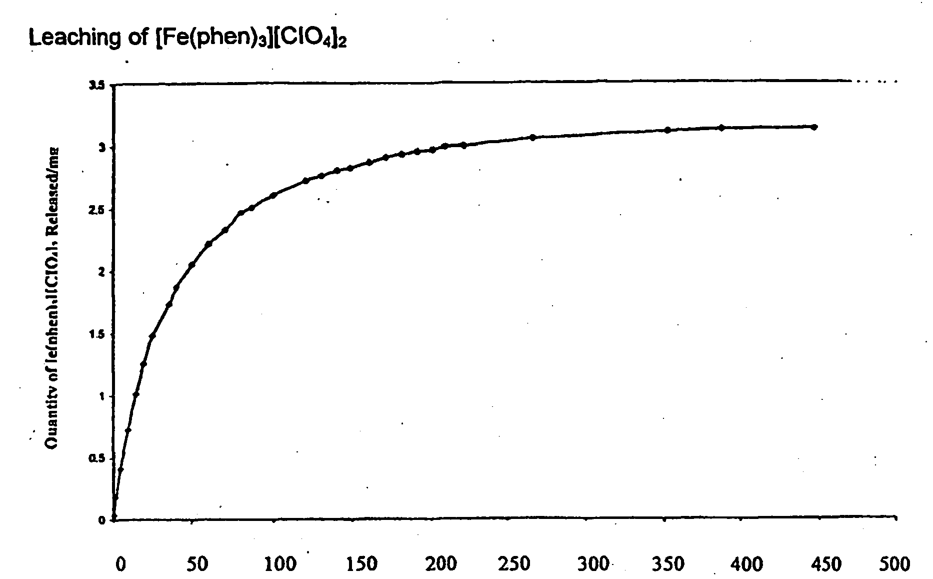

### Leaching of [Fe(phen)₃][ClO₄]₂

Approximately all [Fe(phen)₃][ClO]₂ is released after 300 minutes. Further readings were taken after 24 hours and the observed absorbance decreased. After a further 48 hours most complete disappearance of colour was observed. This may be due to the hydrolysis of the [Fe(phen)₃][ClO]₂ by the HA block.

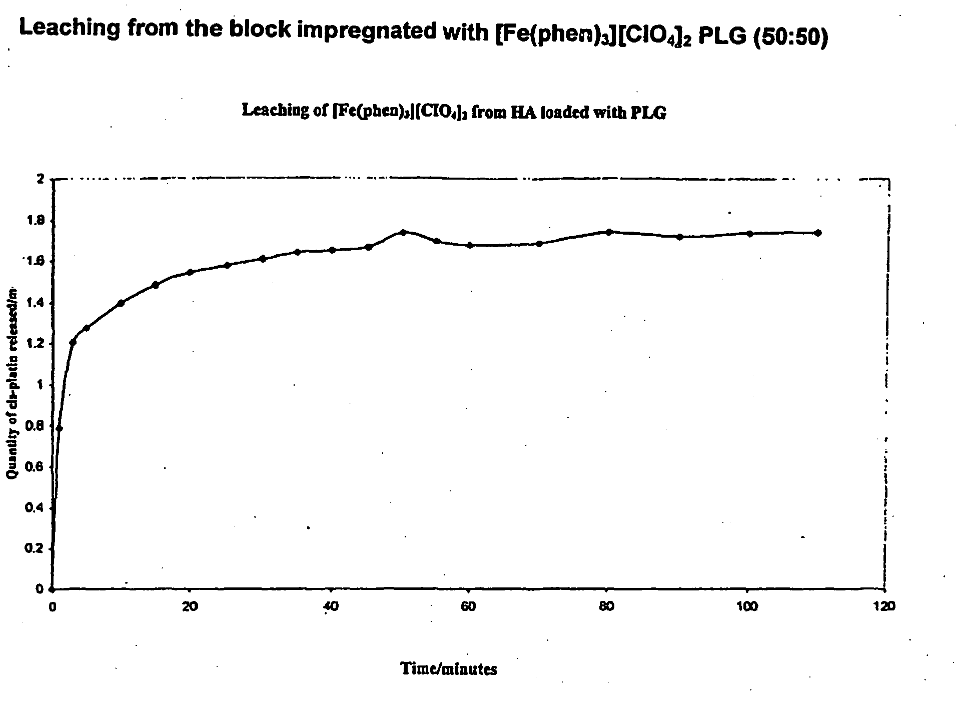

When the block was first immersed in the water bubbles were observed on the surface of the block, in addition to the observed buoyancy this indicates the trapping of air within the block. After 24 hours the block was no longer buoyant which may indicate penetration of the water through the block. The results show that the impregnation of PLG into the block significantly slows the release of Iron Tris Phen. Release of further quantities of complex is observed even after 5 days.

The quantity of [Fe(phen)₃][ClO₄]₂ absorbed onto HA is increased by the application of a vacuum during loading. Increased penetration is observed when acetonitrile is used as the solvent rather than methanol.

The release of [Fe(phen)₃][ClO₄]₂ from HA proceeds by an initial burst (due to release of the complex from the surface of the block) followed by a slower phase (release of complex from within the block). The overall release is fast with the majority of complex released after 300 minutes. The impregnation of HA with a mixture of [Fe(phen)₃][ClO₄]₂ and PLG dramatically slows the release of [Fe(phen)₃][ClO₄]₂ with continuing release of the complex observed even after 5 days.

### EXAMPLE IV

### The impregnation of HA with the anti-cancer drug Cis Platin.

Using vacuum method 3 cis-platin (0.025 g, 0.08mmol) in an aqueous sodium chloride solution (50 cmJ of a 0.9% by weight solution) was injected onto an HA block of porosity 84.04% (0003), dimensions, 21 x 23 x 15 mm, weight 4.2 g. After drying patches of yellow presumed to be cis-platin were observed on the surface of the block. No yellow colour was observed within the block.

### Leaching

Leaching experiments were carried out in distilled water (35 cm³) with stirring, in the absence of light and closed to prevent evaporation. Absorbance was recorded at A 206 nm at appropriate intervals and all caclulations required the extinction coefficient 3057 mol⁻¹ dm³ cm⁻¹.

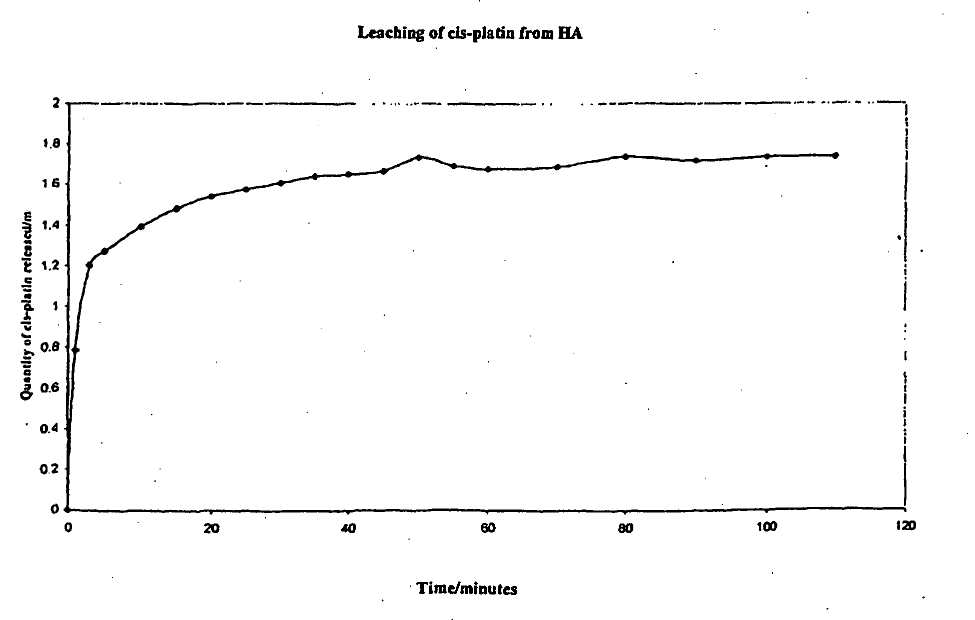

Release of cis-platin is rapid - almost the entire drug released after 45 minutes. The fast release of the drug may indicate that penetration into the block is not occurring and the drug is merely being released from the surface of the block.

### EXAMPLE V

### The use of Poly(DL-Lactide-co-Glycolide) to slow the relase of Cis-Platin

Since PLG is not soluble in water and cis-platin is not soluble in acetonitrile, a two-phase loading programme was undertaken. Using vacuum method 3 cis-platin (0.025 g, 0.08 mmol) was initially loaded onto the block in an aqueous sodium chloride solution (50 cm³ of a 0.9% by weight solution). After drying the block in an oven overnight the vacuum method was again used to inject a solution of PLG (85:15) (0.105 g) in acetonitrile (50 cm³). Again the block was oven dried overnight.

### Conclusion

Release of cis-platin from HA is also fast with the majority of the drug released after only 45 minutes.

### EXAMPLE VI

### Release of the anti-inflammatory agent Prednisolone from HA

Serial dilution experiments were performed to obtain the following calibration chart and equation for Prednisolone.

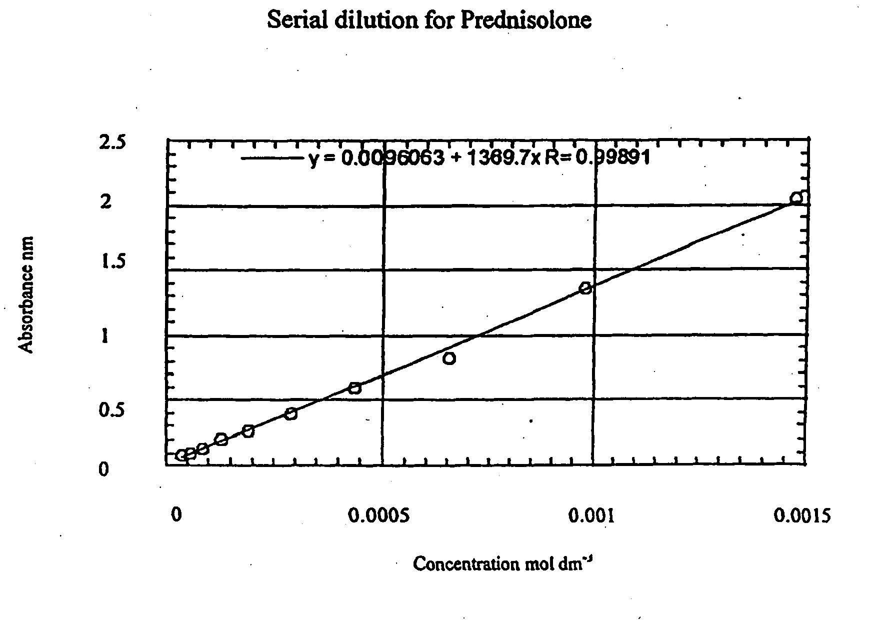

During the preparation of solutions for loading onto the HA discs (this involves stirring the solutions / suspensions of drug / polymer for one hour and occasionally immersion in a sonic tank for 30 seconds) it was discovered that prednisolone dissolves in acetonitrile. It should be made clear therefore that the following technique was not a suspension loaded one.

Four HA discs from the second batch were used for these experiments. Approximately 0.01g of Prednisolone was used in each of the acetonitrile solutions. The experiments were labelled 1AA, 1BB, 1CC and 1DD. 1AA and 1BB were loaded by centrifugation and 1 BB contained 0.1 g of PLG (50:50). 1 CC and 1DD were loaded using the vacuum ' technique and 1DD contained 0.1 g of PLG (50:50). The standard leaching experiments were carried out and the results presented below.

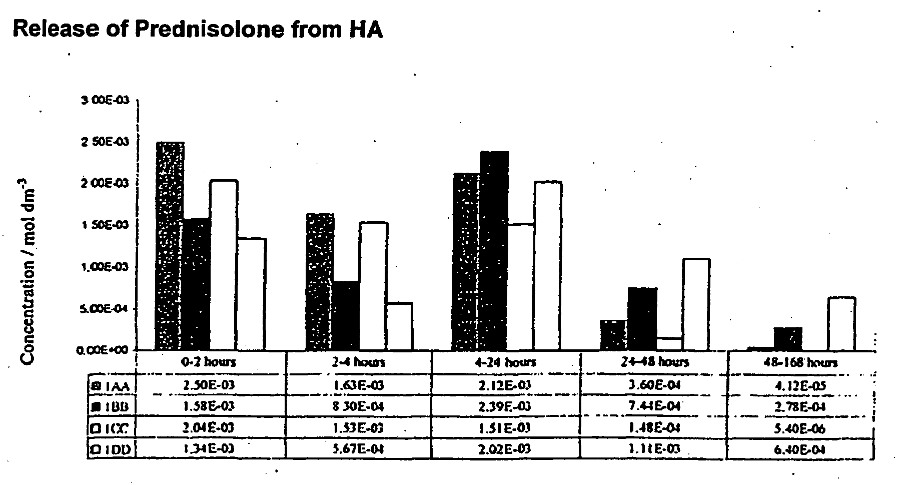

The results show that in the presence of polymer the release of Prednisolone from HA is slowed. The following two graphs show the accumulated release of the drug from HA.

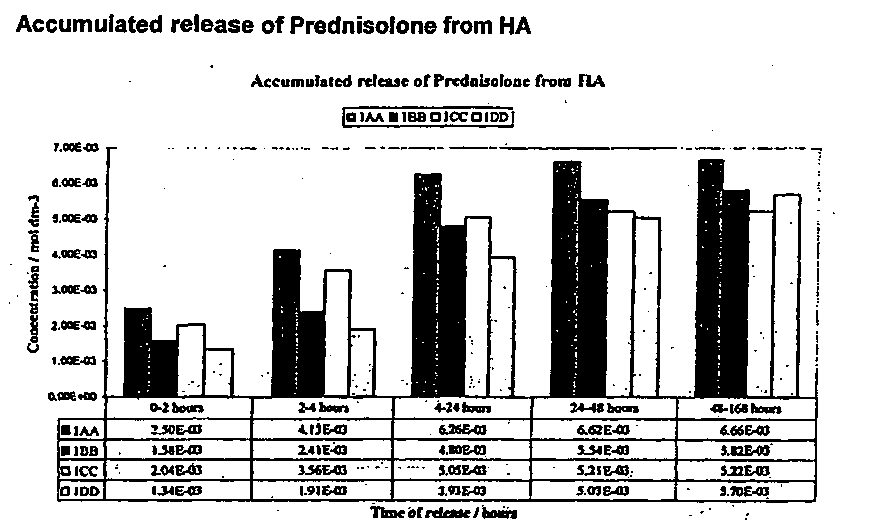

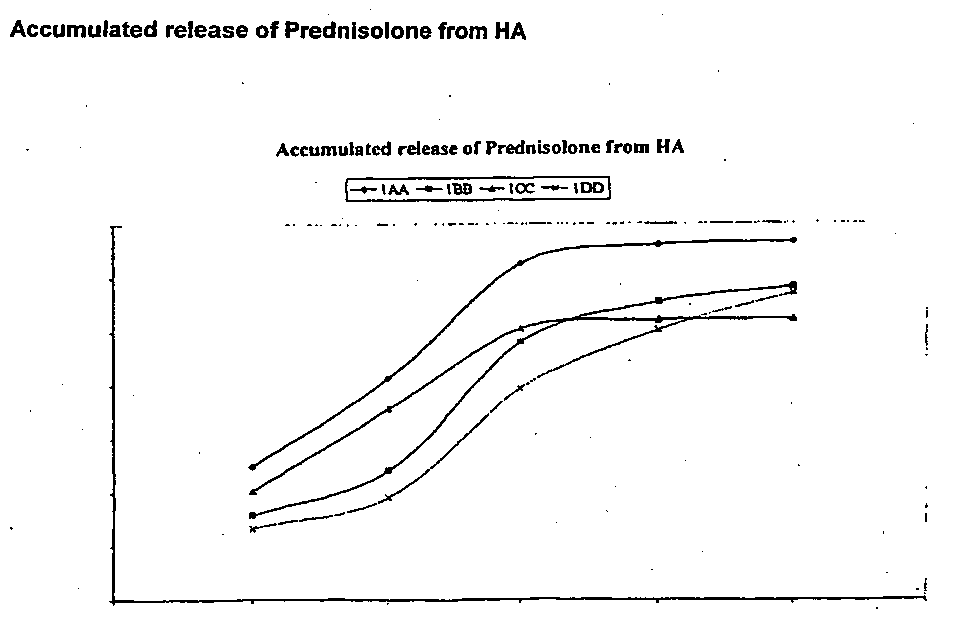

### EXAMPLE VII

### Layering of therapeutic agents in HA discs

Initially the release of MTX from HA using the polymer PLG (75:25) was performed to establish whether this polymer { which is expected to degrade at a slower rate than PLG (50:50)} induced a slowed rate of release of MTX. HA discs from the second batch were used for the experiment and approximately the same quantities of MTX, PLG and acetonitrile were used. The experiment was labelled as follows; IA and IB were loaded by centrifugation and IB contains polymer, IC and ID were loaded by the vacuum technique and ID contains polymer. The following results were obtained.

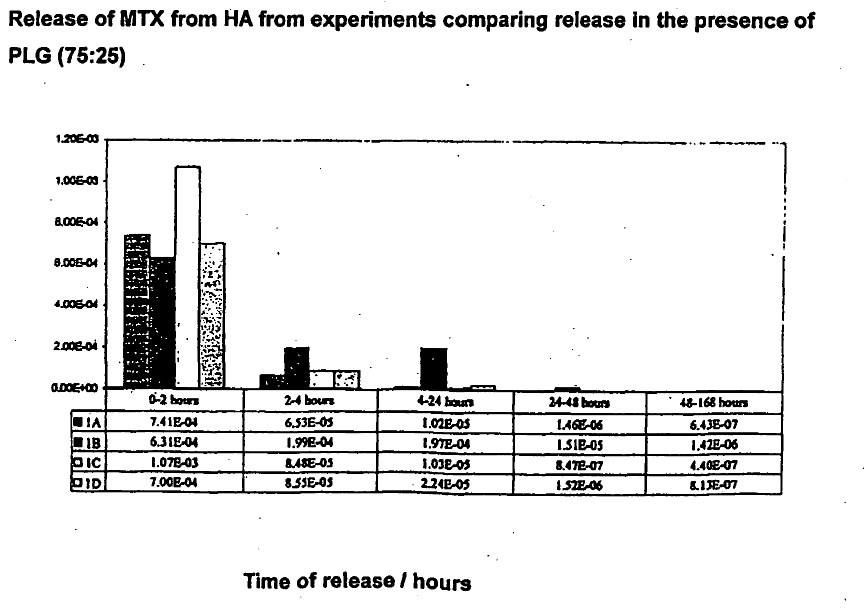

The results confirm that the presence of PLG (75:25) does slow the release of MTX from HA. This effect however does not appear to be great for PLG (75:25) than for PLG (50:50).

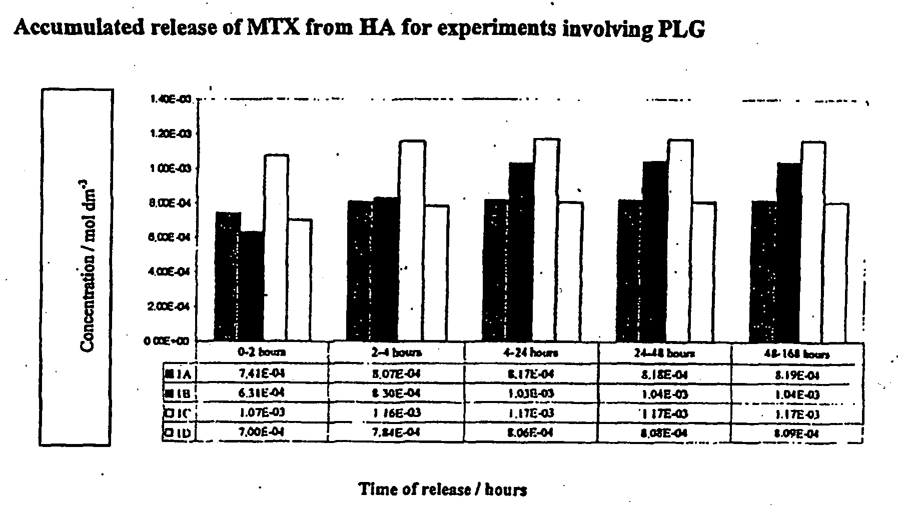

Despite these results indicating that this polymer was not effective in further decreasing the rate of MTX release from HA observation of the discs on conclusion of the experiment indicated that there was still further quantities of MTX trapped onto the disc which were loaded in the presence of polymer. This indicates that further MTX may be released after further polymer degradation.

### EXAMPLE VIII

### Loading of HA with two different drugs incorporated into polymers.

Four HA discs from the second batch were loaded first with MTX suspensions loaded in acetonitrile, two containing the polymer PLG (75:25). Both centrifuge and vacuum techniques were used as described previously. The discs were then allowed to dry in air and subsequently loaded with solutions containing Prednisolone in solution again with two of the solutions containing polymer in this case PLG (50:50). At this point a potential problem was identified, for the two discs loaded in the presence of polymer after loading of Prednisolone the solutions remaining after loading were pale yellow in colour indicating the release of some MTX during the loading process. This is consistent with the second solutions (acetonitrile) dissolving some of the original polymer (PLG (75:25)) on the discs and resulting in the release of some MTX. The leaching from these discs is anticipated to result in the simultaneous release of both drugs. To achieve the effective layering of drugs onto HA two avenues of investigation are proposed. The identification of a variety of biodegradable polymers soluble in different solvents may enable layering of drugs or alternatively the injection of polymer solutions into different areas of HA blocks may prove successful.

### Conclusion

Multiple repeats of MTX release in the presence or absence of PLG (50:50) were not conclusive, probably due to the degradation of MTX and or polymer.

Leaching of MTX (with PLG (50:50) in some discs) from HA with weighting of the discs showed a similar trend as observed for the original experiment with the presence of PLG (50:50) showing a slower release rate of MTX compared to the absence of polymer. At later time periods MTX release in the presence of polymer is greater when compared to discs loaded in the absence of polymer.

Release of the drugs Cis-platin and Prednisolone from HA are also slowed by the presence of PLG (50:50).

Leaching of MTX (with PLG (75:25) in some discs) from HA does not appear to showa slower release rate than for PLG (50:50) however observation of the discs at the conclusion of the leaching experiments does indicate the presence of further quantities of MTX. When a second polymer/drug solution is applied some release of the original drug is observed due to some of the original polymer being dissolved. Biodegradable polymers with different solubilities to facilitate layering or the injection of solutions into HA will overcome this problem.

## Claims

1. A preformed porous ceramic carrier comprising an interconnected skeleton made up of scaffolding and struts and having pores, the majority of which are in the range of from 20 to 1000 micron, the pores comprising a network of coalesced spheres, the carrier having a density of less than about 40% theoretical, the pores containing a drug therein, the rate of release of the drug from the carrier being controlled by having the drug located within the pores in a degradable support.

2. A carrier according to Claim 1, wherein the skeleton has average pore sizes in the range of 20 to 800 micron.

3. A carrier according to Claim 2, wherein the average pore size is in the range of 60 to 800 micron.

4. A carrier according to Claim 3, wherein micropores are present, which micropores being formed by sintering the precursor of the carrier under conditions which were below those required for full sintering.

5. A carrier according to any preceding Claim, wherein the skeleton is a biocompatible material.

6. A carrier according to any preceding Claim, wherein the density ranges from about 10% to about 30% of theoretical density.

7. A carrier according to any preceding Claim, wherein the drug within the pores comprises one or more of:- growth factors; antibiotics; vitamins; proteins; hormones; a chemotherapy agent; or a radio opacifying agent.

8. A carrier according to Claim 7, wherein the pores containing any or more of the following growth factors:
- a bone growth material
- FGF (fibroplast growth factor)
- IGF-1
- IGF-II
- PDGF (platelet derived growth factor)
- TGF-B (transforming growth factor)
- a bone forming or bone degrading cell.
- BMP-Z
- HGH
- Concentrations of human derived growth factors

9. A carrier according to Claim 7, wherein the chemotherapy agent is cisplatin.

10. A carrier according to Claim 7, wherein the radio opacifying agent is strontium -67 or samarium -153.

11. A carrier according to Claim 7, wherein the agent is methotrexate.

12. A carrier according to any preceding Claim, wherein the pores contain one or more of Werner-type co-ordination complexes; macrocylic complexes; metallocenes and sandwich complexes and organometallic.

13. A carrier according to any preceding Claim, wherein the surface of the pores has been modified to control release of the drug.

14. A carrier according to any of Claims 1 to 13, wherein the surface of the pores has been modified by treatment with acid or alkali or plasma or chemical vapour deposition.

15. A carrier according to any of Claims 1 to 13, wherein the degradable support comprises a biodegradable support.

16. A carrier according to Claim 15, wherein the degradable support is a collagen or polymer.

17. A carrier according to Claim 15 or 16, wherein the support is one of poly (carboxyphenoxy) propane sebacic acid (PCPP.SA), precipitated calcium carbonate (PCC), (carboxyphenoxy) propane sebacic acid (CPP.SA), (fatty acid dimer sebacic acid) poly trimethylene carbonate (FAD-SAPTMC), poly(aspartic acid) (PAA).

18. A carrier according to Claim 15, 16 or 17, wherein the pores contain layers of drug or biodegradable support, each layer being different from its neighbour or neighbours.

19. A carrier according to any of Claims 16 to 18, wherein the pores contain material in layers, arranged as alternating layers of drug-free layer and of drug containing layers or by the concentration of drug across different layers of collagen or polymer.

20. A carrier according to any preceding Claim, wherein the drug is introduced into the pores by one or more of a centrifugation, immersion, vacuum impregnation or freeze drying technique.

21. A carrier according to any preceding Claim, wherein the exterior surface has been coated with a biodegradable polymer containing a drug.

22. A carrier according to Claim 1, wherein the skeleton is partially or fully resorbable.

23. A carrier according to Claim 22, wherein the skeleton is formed of calcium phosphate or hydroxyapatite (HA).

24. A carrier according to Claim 1, wherein the drug is Ferrum trisphenanthrolyn perchlorate (II).

25. A carrier according to Claim 24, wherein the Ferrum trisphenanthrolyn perchlorate (II) has been loaded into the pores by vacuum impregnation.

26. A carrier according to Claim 24, wherein the pores further contain glycolide, the rate of release of Ferrum trisphenanthrolyn perchlorate (II) being controlled.

27. A carrier according to Claim 1, wherein the drug comprises Cisplatin and a glycolide, the rate of release of the Cisplatin and a glycolide being controlled.

28. A carrier according to Claim 1, wherein the drug comprises prednisolone, the rate of release of the prednisolone being controlled.

29. A carrier according to any preceding Claim, shaped for orthopaedic, maxillo-facial, or cranio-facial replacement or the like..

30. A carrier according to any of Claims 1 to 28, shaped for location at an intramuscular site, interperitoneal site, subcutaneous site, central nervous system or occular sites.

## Patentansprüche

1. Ein vorgeformter poröser keramischer Träger, umfassend ein in sich verbundenes Gerüst, das aus Gerüstmaterial und Streben aufgebaut ist und das Poren aufweist, wobei die Mehrzahl von diesen in dem Bereich von 20 bis 1000 Mikron liegt, die Poren ein Netzwerk aus verschmolzenen Sphären umfassen, der Träger eine Dichte von weniger als ca. 40% der Theorie aufweist, die Poren darin ein Arzneimittel enthalten, die Freisetzungsrate des Arzneimittels aus dem Träger gesteuert wird, indem das Arzneimittel innerhalb der Poren in einem abbaubaren Trägermaterial untergebracht wird.

2. Ein Träger gemäß Anspruch 1, wobei das Gerüst durchschnittliche Porengrößen in dem Bereich von 20 bis 800 Mikron aufweist.

3. Ein Träger gemäß Anspruch 2, wobei die durchschnittliche Porengröße in dem Bereich von 60 bis 800 Mikron liegt.

4. Ein Träger gemäß Anspruch 3, bei dem Mikroporen vorliegen, wobei die Mikroporen durch Sintern des Vorläufers des Trägers unter Bedingungen, die unterhalb von denen liegen, die für eine vollständige Sinterung benötigt werden, gebildet werden.

5. Ein Träger gemäß irgendeinem vorhergehenden Anspruch, wobei das Gerüst ein biokompatibles Material ist.

6. Ein Träger gemäß irgendeinem vorhergehenden Anspruch, wobei die Dichte von ca. 10% bis ca. 30% der theoretischen Dichte reicht.

7. Ein Träger gemäß irgendeinem vorhergehenden Anspruch, wobei das Arzneimittel innerhalb der Poren eines oder mehrere umfasst von: Wachstumsfaktoren; Antibiotika; Vitaminen; Proteinen; Hormonen; einem Chemotherapiemittel; oder einem strahlenundurchlässig machenden Mittel.

8. Ein Träger gemäß Anspruch 7, wobei die Poren irgendeinen oder mehrere der folgenden Wachstumsfaktoren enthalten:
- ein Knochenwachstumsmaterial
- FGF (Fibroplasten-Wachstumsfaktor)
- IGF-I
- IGF-II
- PDGF (Blutplättchen-Wachstumsfaktor)
- TGF-B (Transformationswachstumsfaktor)
- eine Knochen bildende oder Knochen abbauende Zelle
- BMP-Z
- HGH
- Konzentrationen von vom Menschen stammenden Wachstumsfaktoren.

9. Ein Träger gemäß Anspruch 7, wobei das Chemotherapiemittel Cisplatin ist.

10. Ein Träger gemäß Anspruch 7, wobei das strahlenundurchlässig machende Mittel Strontium -67 oder Samarium -153 ist.

11. Ein Träger gemäß Anspruch 7, wobei das Mittel Methotrexat ist.

12. Ein Träger gemäß irgendeinem vorhergehenden Anspruch, wobei die Poren einen oder mehrere von Koordinations-Komplexen vom Werner-Typ; makrozyklischen Komplexen; Metallocenen und Sandwich-Komplexen und Organometallika (organometallic) enthalten.

13. Ein Träger gemäß irgendeinem vorhergehenden Anspruch, wobei die Oberfläche der Poren modifiziert wurde, um die Freisetzung des Arzneimittels zu steuern.

14. Ein Träger gemäß irgendeinem der Ansprüche 1 bis 13, wobei die Oberfläche der Poren durch Behandlung mit Säure oder Alkali oder Plasma- oder chemische Dampfabscheidung modifiziert wurde.

15. Ein Träger gemäß irgendeinem der Ansprüche 1 bis 13, wobei das abbaubare Trägermaterial ein biologisch abbaubares Trägermaterial umfasst.

16. Ein Träger gemäß Anspruch 15, wobei das abbaubare Trägermaterial ein Kollagen oder Polymer ist.

17. Ein Träger gemäß Anspruch 15 oder 16, wobei das Trägermaterial eines von Poly(carboxyphenoxy)propansebacinsäure (PCPP.SA), ausgefälltem Calciumcarbonat (PCC), (Carboxyphenoxy)propansebacinsäure (CPP.SA), (Fett-säuredimersebacinsäure)polytrimethylencarbonat (FAD-SAPTMC), Poly(asparaginsäure) (PAA) ist.

18. Ein Träger gemäß Anspruch 15, 16 oder 17, wobei die Poren Schichten eines Arzneimittels oder biologisch abbaubaren Trägermaterials enthalten, wobei jede Schicht von ihrem Nachbar oder ihren Nachbarn verschieden ist.

19. Ein Träger gemäß irgendeinem der Ansprüche 16 bis 18, wobei die Poren Material in Schichten enthalten, welches als abwechselnde Schichten von Arzneimittelfreier Schicht und von Arzneimittelenthaltenden Schichten oder durch die Konzentrierung von Arzneimittel über verschiedene Schichten von Kollagen oder Polymer hinweg angeordnet ist.

20. Ein Träger gemäß irgendeinem vorhergehenden Anspruch, wobei das Arzneimittel in die Poren eingeführt wird durch eines oder mehrere von einer Zentrifugation, Immersion, Vakuumimprägnierung oder Gefriertrocknungstechnik.

21. Ein Träger gemäß irgendeinem vorhergehenden Anspruch, wobei die äußere Oberfläche mit einem biologisch abbaubaren Polymer, welches ein Arzneimittel enthält, beschichtet wurde.

22. Ein Träger gemäß Anspruch 1, wobei das Gerüst teilweise oder vollständig resorbierbar ist.

23. Ein Träger gemäß Anspruch 22, wobei das Gerüst aus Calciumphosphat oder Hydroxylapatit (HA) gebildet ist.

24. Ein Träger gemäß Anspruch 1, wobei das Arzneimittel Eisentrisphenanthrolynperchlorat (II) ist.

25. Ein Träger gemäß Anspruch 24, wobei das Eisentrisphenanthrolynperchlorat (II) durch Vakuumimprägnierung in die Poren geladen wurde.

26. Ein Träger gemäß Anspruch 24, bei dem die Poren weiterhin Glycolid enthalten, wobei die Freisetzungsrate von Eisentrisphenanthrolynperchlorat (II) gesteuert wird.

27. Ein Träger gemäß Anspruch 1, bei dem das Arzneimittel Cisplatin und ein Glycolid umfasst, wobei die Freisetzungsraten des Cisplatins und eines Glycolids gesteuert werden.

28. Ein Träger gemäß Anspruch 1, bei dem das Arzneimittel Prednisolon umfasst, wobei die Freisetzungsrate des Prednisolons gesteuert wird.

29. Ein Träger gemäß irgendeinem vorhergehenden Anspruch, welcher für einen orthopädischen, maxillofazialen oder kraniofazialen Ersatz oder dergleichen gestaltet ist.

30. Ein Träger gemäß irgendeinem der Ansprüche 1 bis 28, welcher zur Lokalisierung an einer intramuskulären Stelle, interperitonealen Stelle, subkutanen Stelle, dem zentralen Nervensystem oder okkulären Stellen gestaltet ist.

## Revendications

1. Support en céramique poreux préformé comprenant un squelette interconnecté à base d'une charpente et d'éléments d'étayage d'un échafaudage et d'étais et ayant des pores, dont la majorité se situe dans la plage de 20 à 1000 microns, ces pores comprenant un réseau de sphères coalescées, le support ayant une densité de moins d'environ 40 % de la densité théorique, les pores incluant un médicament, la vitesse de libération du médicament du support étant contrôlée en ayant le médicament localisé à l'intérieur des pores dans un support dégradable.

2. Support selon la revendication 1, dans lequel le squelette a des tailles de pore moyennes dans la plage de 20 à 800 microns.

3. Support selon la revendication 2, dans lequel la taille moyenne de pore se situe dans la plage allant de 60 à 800 microns.

4. Support selon la revendication 3, dans lequel sont présents des micropores, lesquels micropores sont formés par frittage du précurseur du support dans des conditions inférieures à celles nécessaires pour un frittage complet.

5. Support selon l'une quelconque des revendications précédentes, dans lequel le squelette est un matériau biocompatible.

6. Support selon l'une quelconque des revendications précédentes, dans lequel la densité se situe dans la plage allant d'environ 10 à environ 30 % de la densité théorique.

7. Support selon l'une quelconque des revendications précédentes, dans lequel le médicament dans les pores comprend un ou plusieurs compris parmi les suivants : les facteurs de croissance ; les antibiotiques ; les vitamines ; les protéines ; les hormones ; un agent chimiothérapeutique ; ou un agent radio opacifiant.

8. Support selon la revendication 7, dans lequel les pores contiennent un ou plusieurs parmi les facteurs de croissance parmi les suivants :
- un matériau de croissance osseuse
- le FGF (facteur de croissance fibroblastique)
- l'IGF-1
- l'IGF-II
- le PDGF (facteur de croissance dérivé de la plaquette)
- le TGF-B (facteur de croissance transformant)
- une cellule formant l'os ou dégradant l'os
- le BMP-Z
- le HGH
- des concentrations de facteurs de croissance dérivés de l'humain

9. Support selon la revendication 7, dans lequel l'agent chimiothérapeutique est le cisplatine.

10. Support selon la revendication 7, dans lequel l'agent radio opacifiant est le strontium -67 ou le samarium -153.

11. Support selon la revendication 7, dans lequel l'agent est le méthotrexate.

12. Support selon l'une quelconque des revendications précédentes, dans lequel les pores contiennent un ou plusieurs parmi les complexes de coordination de type Wemer ; les complexes macrocycliques ; les métallocènes et les complexes en sandwich et organométalliques.

13. Support selon l'une quelconque des revendications précédentes, dans lequel la surface des pores a été modifiée pour contrôler la libération du médicament.

14. Support selon l'une quelconque des revendications 1 à 13, dans lequel la surface des pores a été modifiée par le traitement avec un acide ou un alcali ou du plasma ou un dépôt de vapeur chimique.

15. Support selon l'une quelconque des revendications 1 à 13, dans lequel le support dégradable comprend un support biodégradable.

16. Support selon la revendication 15, dans lequel le support dégradable est un collagène ou un polymère.

17. Support selon la revendication 15 ou 16, dans lequel le support est un parmi l'acide sébacique poly (carboxyphénoxy) propane (PCPP.SA), le carbonate de calcium précipité (PCC), l'acide sébacique (carboxyphénoxy) propane (CPP.SA), le carbonate poly triméthylène (acide sébacique de dimère d'acide gras) (FAD-SAPTMC), le poly(acide aspartique) (PAA).

18. Support selon la revendication 15, 16 ou 17, dans lequel les pores contiennent des couches de médicament ou de support biodégradable, chaque couche étant différente de sa (ou de ses) voisine(s).

19. Support selon l'une quelconque des revendications 16 à 18, dans lequel les pores contiennent du matériau en couches, disposées en couches alternées de couche sans médicament et de couches contenant un médicament ou par la concentration du médicament à travers différentes couches de collagène ou de polymère.

20. Support selon l'une quelconque des revendications précédentes, dans lequel le médicament est introduit dans les pores par une ou plusieurs techniques de centrifugation, d'immersion, d'imprégnation sous vide ou de lyophilisation.

21. Support selon l'une quelconque des revendications précédentes, dans lequel la surface extérieure a été revêtue avec un polymère biodégradable contenant un médicament.

22. Support selon la revendication 1, dans lequel le squelette est partiellement ou entièrement résorbable.

23. Support selon la revendication 22, dans lequel le squelette est formé de phosphate de calcium ou d'hydroxyapatite (HA).

24. Support selon la revendication 1, dans lequel le médicament est le perchlorate de trisphénanthroline ferreux (II).

25. Support selon la revendication 24, dans lequel le perchlorate de trisphénanthroline ferreux (II) a été chargé dans les pores par imprégnation sous vide.

26. Support selon la revendication 24, dans lequel les pores contiennent en outre un glycolide, la vitesse de libération du perchlorate de trisphénanthroline ferreux (II) étant contrôlée.

27. Support selon la revendication 1, dans lequel le médicament comprend du cisplatine et un glycolide, la vitesse de libération du cisplatine et d'un glycolide étant contrôlée.

28. Support selon la revendication 1, dans lequel le médicament comprend de la prednisolone, la vitesse de libération de la prednisolone étant contrôlée.

29. Support selon l'une quelconque des revendications précédentes, formé pour le remplacement orthopédique, maxillo-facial ou cranio-facial ou similaire.

30. Support selon l'une quelconque des revendications 1 à 28, formé pour l'emplacement à un site intramusculaire, un site interpéritonéal, un site sous-cutané, le système nerveux central ou des sites oculaires.
